# EUROPÄISCHE PATENTSCHRIFT

(11) **EP 2 822 476 B1**
(45) Veröffentlichungstag und Bekanntmachung des Hinweises auf die Patenterteilung: **18.07.2018**
(21) Anmeldenummer: 13709856.2
(22) Anmeldetag: 07.03.2013
(51) Int. Cl.: A61B 17/02

(54) **DOPPELVALVE UND DAZU PASSENDER CHIRURGISCHER RETRAKTOR**
DOUBLE VALVE AND CORRESPONDING SURGICAL RETRACTOR
DOUBLE VALVE ET ÉCARTEUR CHIRURGICAL ADAPTÉ À CELLE-CI

(30) Priorität: 09.03.2012 DE 102012004555
(43) Veröffentlichungstag der Anmeldung: 14.01.2015
(73) Patentinhaber: Aesculap AG, 78532 Tuttlingen (DE)
(72) Erfinder: BECK, Thomas, 78591 Durchhausen (DE); MORALES, Pedro, 78532 Tuttlingen (DE); HUBER, Christian, 78570 Mühlheim (DE)
(74) Vertreter: Koller, Tobias Kilian
(86) Internationale Anmeldenummer: PCT/EP2013/054668
(87) Internationale Veröffentlichungsnummer: WO 2013/132039

(56) Entgegenhaltungen:
- DE-A1- 3 509 787
- DE-A1-102006 036 117
- DE-U1-202011 051 999
- US-A1- 2010 286 485
- US-A1- 2011 144 450

## Beschreibung

Die vorliegende Erfindung betrifft eine Doppelvalve für einen chirurgischen Retraktor sowie einen chirurgischen Retraktor mit einer Doppelvalve. Bei der Herzchirurgie wird für gewöhnlich das komplette Sternum geöffnet um am oder im Herzen operieren zu können. Durch diese Operationsmethode entstehen für den Patienten ein großes Trauma und eine lange Erholungszeit. Dies ruft häufig Probleme mit Infektionen und bei der Heilung hervor. Aus diesem Grund entwickeln Ärzte neue minimal invasive Operationstechniken. Eine Beispiel für solch eine minimal invasive Operationstechnik ist die sogenannte MIDCAB Operation (Minimally Invasive Direct Coronary Artery Bypass bzw. Minimal Invasive Direkte Coronar-Arterielle Bypassoperation). Bei dieser Operation erfolgt der Zugang zum Operationsgebiet nicht durch das Sternum sondern seitlich durch die Rippen des Patienten. Die Rippen des Patienten müssen hierzu aufgespreizt werden, um dem Operateur einerseits einen Zugang zum Operationsgebiet zu bieten und ihm andererseits einen Blick auf bzw. in das Operationsgebiet zu ermöglichen. Für das Spreizen der Rippen ist ein chirurgischer Retraktor mit entsprechenden Valven bzw. Valvenelementen erforderlich.

### Stand der Technik

Gewöhnliche Valven, wie sie in der Sternumschirurgie eingesetzt werden, sind für ein Aufbiegen der Rippen ungeeignet, da die Form der Valven eben und gerade ist. Die anatomischen Gegebenheiten der Rippen im Bereich des seitlichen Brustkorbes sind jedoch anders als im Bereich des Sternums. Das Sternum wird gewöhnlich durch einen möglichst geraden Schnitt geöffnet. Daher sind die Anlageflächen am Sternum meist ebene Flächen und ein Retraktor für die Sternumschirurgie verfügt über Valven, die zur Anlage an einer im Wesentlichen ebenen Anlagefläche geeignet sind. Im Gegensatz dazu sind die Rippen eines Patienten in einer Seitenansicht gebogen. Wird der Zugang zum Operationsgebiet zwischen zwei benachbarten Rippen eingerichtet, bildet die obere Rippe eine konvexe Anlagefläche und die untere Rippe eine konkave Anlagefläche für die Valven des chirurgischen Retraktors. Bei der Verwendung eines Retraktors mit je einer gewöhnlichen Valve aus dem Bereich der Sternumschirurgie führt dies dazu, dass die gesamte Spreizkraft des Retraktors an der unteren Rippe an nur zwei Punkten eingeleitet wird, nämlich den beiden seitlichen Rändern der Valve, und an der oberen Rippe sogar nur an einem einzigen Punkt, nämlich etwa in der Mitte der Valve. Durch diese punktuelle Krafteinleitung der Spreizkraft des Retraktors in die Rippen des Patienten können unbeabsichtigte Rippenbrüche oder Knocheneinbrüche bzw. Knochenabsplitterungen hervorgerufen werden. Die vorliegende Erfindung soll diese Rippenbrüche vermeiden und so die Erholungszeit des Patienten noch weiter verringern.

Im Stand der Technik sind verschiedenste chirurgische Retraktoren bekannt. In dem Dokument US 2011/0172494 ist ein chirurgischer Retraktor gezeigt, der einen seitlichen Zugang zum Brustraum des Patienten ermöglicht. Dieser chirurgische Retraktor verfügt über zwei gebogene Valven, genauer gesagt über eine konkave Valve auf der einen Seite und auf der gegenüberliegenden Seite über eine konvexe Valve. Durch die Krümmung bzw. Biegung der beiden Valven des Retraktors ist somit direkt festgelegt, wie der Retraktor am Patienten einzusetzen ist. Dabei muss die konkave Valve an der konvexen, oberen Rippe in Anlage gebracht werden und die konvexe Valve muss an der konkaven, unteren Rippe in Anlage gebracht werden. Da die Biegung bzw. Krümmung der Valven aber nicht besonders stark ausgeprägt ist, besteht hier die Gefahr der Verwechslung der beiden Valvenkrümmungen.

US2010286485 A1 offenbart einen Retraktor mit einem Grundelementen welches zwei Valven trägt. DE202011051999 U1 offenbart ein Retraktionssystem, insbesondere zum Spreizen eines durchtrennten Sternums, mit einer Haltevorrichtung, zwei Spreizarmen, welch letztere jeweils mit einem ersten Ende an der Haltevorrichtung in einem in einer Spreizebene veränderlichen Abstand voneinander gehalten sind, sowie mindestens zwei Rückhalteelementen, die an den zu retrahierenden Knochenmaterialien angreifen und diese halten. Die Aufgabe der vorliegenden Erfindung ist es, eine Möglichkeit zu schaffen, einen seitlichen Zugang zum Brustraum eines Patienten herzustellen, bei dem eine Vertauschung der Valven ohne Folgen bleibt.

Die Aufgabe der vorliegenden Erfindung wird gelöst durch eine Doppelvalve nach Anspruch 1. Weitere vorteilhafte Fortbildungen sind Gegenstand der abhängigen Ansprüche.

Gemäß einem ersten Aspekt der vorliegenden Erfindung ist eine Doppelvalve für einen chirurgischen Retraktor offenbart mit einem Grundkörper, der einen im Wesentlichen zylindrischen Montagedorn zur Montage der Doppelvalve an einem chirurgischen Retraktor aufweist, und mindestens zwei Valvenelementen, die so an dem Grundkörper angebracht sind, dass sie zumindest in einem gewissen Winkelbereich gegenüber dem Grundkörper drehbar und/oder verschiebbar sind.

Hierbei können die Valvenelemente gegen eine Federlast verschiebbar sein. Insbesondere können sie dann, wenn mehr als zwei Valvenelemente an einem Grundkörper vorgesehen sind, so zueinander verschiebbar sein, dass sie sich an eine bogenförmige Rippe anpassen können, d.h. eine konkave oder konvexe Anordnung annehmen. Wenn die Doppelvalve an einem Retraktor montiert ist und dieser am Patienten zum Einsatz kommt, indem er zwischen zwei Rippen des Patienten eingesetzt ist, entspricht diese Verschiebbarkeit einer Richtung kranial-kaudal bzw. oben-unten. Sind die Valvenelemente einer Doppelvalve im Wesentlichen nebeneinander angeordnet, so entspricht diese Richtung in Bezug auf die Doppelvalve einer vorn-hinten Richtung, wobei vorn die Richtung zu der Inzision hin beschreibt. Insofern ist es besonders vorteilhaft, wenn die Valvenelemente gegen eine Federkraft von einer Grundstellung nach vorn verschiebbar sind.

Sind nur zwei Valvenelemente vorgesehen, dann passen sich diese durch ihre Drehbarkeit automatisch an die Form der jeweiligen Rippe an. Bei einer konkaven Rippe, d.h. bei der unteren Rippe, drehen sich die beiden Valvenelemente leicht zueinander hin, bei einer konvexen Rippe, d.h. bei der oberen Rippe, drehen sie sich leicht voneinander weg. Sind mehr als zwei Valvenelemente an einem Grundkörper vorgesehen, muss bei einer konvexen Rippe zumindest das mittlere Valvenelement bzw. die mittleren Valvenelemente in vorn-hinten Richtung verschiebbar sein, sodass sich auch in diesem Fall alle Valvenelemente durch eine Drehung an die Form der Rippe anpassen können. Im Falle einer konkaven Rippe müssen alle Valvenelemente bis auf das mittlere bzw. die beiden mittleren Valvenelemente in der vorn-hinten Richtung verschiebbar sein, um solche eine Anpassung zu ermöglichen. Vorteilhafter Weise sind dann aber alle Valvenelemente verschieblich an dem Grundkörper angebracht. Eine vollständige Drehung der Valvenelemente um 360° ist nicht erforderlich, kann aber umgesetzt werden. Entscheidend ist, dass der Winkelbereich ausreichend groß ist, um eine Anpassung an die Konvexität und Konkavität verschiedener Rippen unterschiedlicher Patienten zu ermöglichen. Dazu muss der Winkelbereich nicht allzu groß sein.

Der Montagedorn ist so gestaltet, dass er in einer entsprechenden Aufnahme in einem Retraktorarm aufgenommen werden kann und dort gegen ein Herausfallen gesichert werden kann. Eine gleichartige Montage kann auch an einem Verbindungselement erfolgen.

Gemäß der vorliegenden Erfindung ist der Montagedorn so gestaltet, dass dann, wenn die Doppelvalve an einem chirurgischen Retraktor montiert ist, der Grundkörper der Doppelvalve zumindest in einem vorgegebenen Winkelbereich gegenüber dem chirurgischen Retraktor drehbar ist.

Mit der Drehbarkeit des Grundkörpers gegenüber dem chirurgischen Retraktor passt sich die Lage der gesamten Doppelvalve mit ihren mindestens zwei Valvenelementen an die geometrischen Verhältnisse der Anlagestelle an, sodass keine Momente an den Arm des Retraktors übertragen werden und auch keine Momente von dem Arm des Retraktors an die Doppelvalve übertragen werden, welche dann zu einer übermäßigen Einwirkung eines Valvenelements auf dessen Anlagestelle führen könnte. Solch eine übermäßige Einwirkung könnte ansonsten zu einem unnötigen lokalen Trauma an der Anlagestelle führen.

Gemäß einer weiteren vorteilhaften Weiterentwicklung des ersten Aspekts der vorliegenden Erfindung weist der Montagedorn eine zumindest teilweise umlaufende Befestigungsnut auf.

Mit Hilfe der zumindest teilweise umlaufenden Befestigungsnut kann eine formschlüssige Verbindung zwischen der Doppelvalve und einem Arm eines Retraktors hergestellt werden, ohne dass die Drehbarkeit der Doppelvalve bzw. des Grundkörpers gegenüber dem Retraktorarm aufgegeben werden muss. In die Befestigungsnut kann z.B. mindestens eine federbelastete Kugel eingreifen und so eine formschlüssige Hinterschneidung herstellen. Zusätzlich kann die Hinterschneidung gegen ein versehentliches Lösen der Hinterschneidung und somit ein unbeabsichtigtes Lösen der Doppelvalve von dem Retraktorarm durch einen Sicherungsring oder dergleichen gesichert sein, wie dies häufig bei Druckleitungen praktiziert wird.

Gemäß einer weiteren vorteilhaften Weiterentwicklung des ersten Aspekts der vorliegenden Erfindung ist die Tiefe der Befestigungsnut entlang ihrer Umlaufrichtung veränderlich.

Dies sorgt in Verbindung mit einer Hinterschneidung mit einer einzelnen federbelasteten Kugel dafür, dass sich die Doppelvalve so ausrichtet, dass sich die Feder, welche die Kugel in die Befestigungsnut drängt, maximal entspannt. Dies bedeutet, dass sich der Grundkörper einer lastfreien Doppelvalve in eine Stellung zurückdreht, in der die Kugel an der tiefsten Stelle der Nut anliegt. Dazu muss sich die Tiefe der Nut kontinuierlich bzw. stetig verändern und darf keine Vielzahl von lokalen Tiefstpunkten aufweisen. Sinnvollerweise ist diese Stellung, in welche sich der Grundkörper der Doppelvalve zurückdreht, die Grundstellung der Doppelvalve, in der die Verbindungslinie der Befestigungspunkte der Valvenelemente an dem Grundkörper im Wesentlichen parallel zu der Anlagefläche an der Körperöffnung des Patienten verläuft. Üblicherweise steht diese Verbindungslinie in der Grundstellung auch senkrecht auf dem Abstandsregelelement des Retraktors.

Gemäß der vorliegenden Erfindung weisen die Valvenelemente ein proximales Ende, an dem sie in dem Grundkörper aufgenommen sind und an diesem drehbar befestigt sind, und ein freies distales Ende auf.

Wenn die proximalen Enden der Valvenelemente in Aussparungen aufgenommen sind, welche in dem Grundkörper ausgebildet sind, können die Seitenwände dieser Aussparungen die Drehung der Valvenelemente gegenüber dem Grundkörper begrenzen. Dies ist sinnvoll, da eine Drehbarkeit der Valvenelemente um 360° nicht brauchbar ist, sondern im Gegensatz dazu beim Einsetzen des Retraktors in die aufzuspreizende Körperöffnung des Patienten in eine falsche Stellung geraten können und somit das Einführen des Retraktors behindern können. Die Valvenelemente können auch in einer gemeinsamen Aussparung aufgenommen sein, sodass nur eine äußere Seitenwand existiert, die ein Verdrehen des jeweiligen Valvenelements nach außen begrenzt. Die proximalen Enden der Valvenelemente müssen aber nicht in dem Grundkörper aufgenommen sein, sondern können, in alternativen Ausführungen die nicht unter den Patentanspruch fallen, auch an der Oberseite oder der Unterseite des Grundkörpers angebracht sein.

Gemäß einer weiteren vorteilhaften Weiterentwicklung des ersten Aspekts der vorliegenden Erfindung weist der Grundkörper in dem Bereich, in dem die proximalen Enden der Valvenelemente aufgenommen sind, Reinigungsöffnungen auf, um ein korrektes Reinigen und Sterilisieren der Doppelvalve sicher zu stellen.

Bei der Verwendung der Doppelvalve können Blut, Gewebeteile, Knochensplitter oder andere Verschmutzungen in die Öffnungen eintreten, in denen die Valvenelemente aufgenommen sind. Diese sind nur sehr schwer wieder zu entfernen, wenn diese Öffnungen als Sacklöcher ausgebildet sind. Mit den Reinigungsöffnungen ist es möglich, ein Spülfluid wie beispielsweise Druckluft, Wasser, eine wässrige Seifenlauge oder eine andere Reinigungsflüssigkeit durch die Reinigungsöffnungen gegebenenfalls mit Druck einzuführen und somit grobe und feine Verschmutzungen auszuspülen. Dies erleichtert den Reinigungsvorgang erheblich. Vorteilhafter Weise sind die Reinigungsöffnungen an den Bodenflächen der Sacklöcher ausgebildet, sie können aber auch an der Oberseite, der Unterseite oder den Seitenflächen des Grundkörpers vorgesehen sein. Wichtig ist, dass Sie eine Verbindung zwischen dem jeweiligen Sackloch (d.h. der Öffnung, in der mindestens ein Valvenelement aufgenommen ist) und der Umgebung des Grundkörpers herstellen. Es kann auch mindestens eine Reinigungsöffnung geben, die verschiedene Sacklöcher miteinander verbindet.

Gemäß einer weiteren vorteilhaften Weiterentwicklung des ersten Aspekts der vorliegenden Erfindung weist mindestens ein Valvenelement eine quer zur Längsachse des Valvenelements konvex gekrümmte Innenfläche auf.

Mit einer konvex gekrümmten Innenfläche eines Valvenelements oder der Valvenelemente kann die Doppelvalve während des Einsetzvorgangs in die Körperöffnung des Patienten entlang der Anlagefläche verschoben werden, ohne dass sich ein seitlicher Rand des Valvenelements mit der Anlagefläche verkeilt und bei einem weiteren Verschieben der Doppelvalve entlang der Anlagefläche so verdreht, dass sie mit einer seitlichen Stirnseite an der Anlagefläche anliegt, was zu einem verstärkten Trauma oder gar Knochenausbrüchen an der Anlagefläche führen kann.

Gemäß einer weiteren vorteilhaften Weiterentwicklung des ersten Aspekts der vorliegenden Erfindung weist mindestens ein Valvenelement ein zur Innenseite der Valve gebogenes freies Ende auf.

Dieses zur Innenseite gebogene freie Ende sorgt für einen optimalen Sitz der Doppelvalve bzw. der Valvenelemente an den aufzubiegenden Knochen in der Anlagefläche und verhindert ein Abrutschen der Doppelvalve von den Knochen. Dabei liegt der aufzubiegende Knochen genau an der Innenfläche der Biegung des freien distalen Endes der Valvenelemente an.

Gemäß einer weiteren vorteilhaften Weiterentwicklung des ersten Aspekts der vorliegenden Erfindung ist zwischen mindestens einem Valvenelement und dem Grundkörper ein elastisches Element vorgesehen, welches das mindestens eine Valvenelement in eine vordefinierte Grundstellung drängt.

Auf diese Weise wird verhindert, dass ein Valvenelement beim Einsetzen eines Retraktors mit einer erfindungsgemäßen Doppelvalve ein Valvenelement mit seiner Rückseite, also seiner Außenfläche, an der Anlagefläche in Anlage gerät. Dies würde den Einsetzvorgang behindern und verlangsamen. Das elastische Element muss dabei nicht besonders stark ausgebildet sein.

Gemäß einer weiteren vorteilhaften Weiterentwicklung des ersten Aspekts der vorliegenden Erfindung weist mindestens ein Valvenelement eine weiche Beschichtung oder Polsterung an seiner Innenfläche auf.

Eine solche weiche Beschichtung oder Polsterung verteilt den Anpressdruck, welche von den einzelnen Valvenelementen auf die entsprechenden Stellen der Anlagefläche aufgebracht wird und verringert somit die auftretenden Traumata in der Anlagefläche. Dies sorgt für insgesamt geringere Traumata, geringere Schmerzentwicklung beim Patienten nach der Operation und eine schnellere und bessere Heilung der Wunde.

Gemäß einer weiteren vorteilhaften Weiterentwicklung des ersten Aspekts der vorliegenden Erfindung ist die Verdrehung mindestens eines Valvenelements gegenüber dem Grundkörper begrenzt.

Dazu ist eine Art Anschlag vorgesehen, an welchem das Valvenelement nach Absolvieren einer Drehung um einen bestimmten Winkel in Anlage gerät. Dieser Anschlag kann im Bereich der drehbaren Befestigung des Valvenelements an dem Grundkörper vorgesehen sein. Alternativ dazu kann auch eine Seitenfläche eines Valvenelements an einem solchen Anschlag in Anlage geraten. Dies ist vorteilhaft, da eine Drehung der Valvenelemente nur in einem gewissen und relativ kleinen Winkelbereich Vorteile bringt und eine übermäßige Verdrehung der Valvenelemente gegenüber dem Grundkörper ein Einsetzen des Retraktors behindern kann.

Gemäß einer weiteren vorteilhaften Weiterentwicklung des ersten Aspekts der vorliegenden Erfindung ist die Längsachse jedes Valvenelements um einen Winkel α zwischen 50° und 90°, vorzugsweise zwischen 60° und 80°, und insbesondere zwischen 65° und 75° gegenüber der Ebene des Grundkörpers geneigt.

Die Anlagefläche besteht nicht ausschließlich aus Knochenmaterial. Das Knochenmaterial bildet den am weitesten innen liegenden Bereich der Anlagefläche. Dort liegt das distale Ende der Valvenelemente an. Über dem Knochenmaterial befindet sich eine mehr oder weniger dicke Schicht Weichgewebe. Die Dicke der Weichgewebeschicht hängt ab von der körperlichen Verfassung des Patienten und von dem Ort des Eingriffs am Patienten. Beträgt der Winkel α 90°, so wird das Weichgewebe genauso weit nach außen gedrängt wie die Knochen aufgebogen werden. Bei kleinerem Winkel wird das Weichgewebe weiter nach außen gedrängt als die Knochen aufgebogen werden. Dies führt zu einer Art Trichter im Bereich der Wundöffnung. Aufgrund des Trichters verbessert sich die Sicht des Operateurs in den Patienten hinein. Bei zu kleinem Winkel α von unter 50° droht ein Abrutschen der Valvenelemente von den Knochen, was unbedingt zu verhindern ist, insbesondere während der Operation. Ein vorteilhafter Winkelbereich liegt zwischen 60° und 80°, da dort ein fester Sitz des Retraktors in der aufzuspreizenden Körperöffnung und gleichzeitig eine gute Sicht für den Operateur gewährleistet ist. Im Bereich von 65° und 75° ist der Sitz der Valvenelemente an den Knochen noch besser und die Sicht für den Operateur nach wie vor sehr gut.

Gemäß einem zweiten Aspekt der vorliegenden Erfindung ist eine Valvenanordnung für einen chirurgischen Retraktor offenbart mit einem Verbindungselement, das einen im Wesentlichen zylindrischen Montagedorn und zwei Montageaufnahmen aufweist. Eine Doppelvalve nach einem der vorstehenden Ausführungsbeispiele ist an einer Montageaufnahme des Verbindungselements montiert. An der anderen Montageaufnahme des Verbindungselements ist entweder eine weitere Doppelvalve oder aber eine Einzelvalve montiert.

Mit diesem zweiten Aspekt kann die Funktionsweise der vorstehenden Doppelvalve für größere Körperöffnungen verwendet werden. Der Montagedorn entspricht dabei dem Montagedorn des Grundbauteils der Doppelvalve und die Montageaufnahmen entsprechen den Montageaufnahmen an dem Retraktorarm. Verbindungselemente können in verschiedenen Längen ausgebildet sein und die Valvenanordnung kann nach Bedarf zusammengebaut werden.

Gemäß einem dritten Aspekt der vorliegenden Erfindung ist eine Valvengruppe offenbart, welche mindestens ein Verbindungselement aufweist, das einen im Wesentlichen zylindrischen Montagedorn und zwei Montageaufnahmen aufweist. An den beiden Montageaufnahmen jedes Verbindungselements ist entweder ein weiteres Verbindungselement, eine Valvenanordnung entsprechend dem zweiten Aspekt der Erfindung, eine Doppelvalve entsprechend dem ersten Aspekt der Erfindung oder eine Einzelvalve montiert.

Mit diesem Aufbau kann die Funktionsweise des zweiten Aspekts der vorliegenden Erfindung noch umfassender eingesetzt werden. Die durch den Retraktor auf die Anlagefläche aufgebrachte Kraft kann so auf eine Vielzahl von Lasteinleitungsstellen verteilt werden. Durch eine entsprechende Ausbildung der Valvengruppe kann eine gleichmäßige Last auf alle Valvenelemente verteilt werden. Dazu sind die Hebelarme und die an den Hebeln vorhandene Anzahl von Valvenelementen in eine konstante Beziehung zu setzen. Je geringer die Anzahl von Valvenelementen ist, die sich auf einer Seite des Hebels befinden, desto länger muss der Hebelarm sein, um eine gleichmäßige Krafteinleitung über alle Valvenelemente zu erzielen.

Gemäß einem vierten Aspekt der vorliegenden Erfindung ist eine Doppelvalve nach einem der vorstehenden Aspekte offenbart, wobei die Einzelvalven und/ oder Valvenelemente an ihrem freien Ende so zu der Innenseite der Einzelvalven bzw. Valvenelemente gebogen sind, dass die distalen Biegestellen auf einer Geraden oder einem Bogen liegen.

Mit einer solchen Anordnung kann sichergestellt werden, dass die alle Valvenelemente fest an dem aufzubiegenden Knochen anliegen und nicht zu viel Last auf einzelne Lasteinleitungsstellen aufgebracht wird. Wenn die Biegestellen auf einer geraden liegen, eignet sich die Vorrichtung für eine im Wesentlichen gerade Körperöffnung, beispielsweise an einem Sternum. Liegen die Biegestellen auf einem Bogen, sollte dieser Bogen an die Biegung der aufzubiegenden Knochen angepasst sein. Eine solche Vorrichtung eignet sich zum Beispiel für den seitlichen Rippenbereich.

Gemäß einem fünften Aspekt der vorliegenden Erfindung ist ein chirurgischer Retraktor offenbart mit einer Doppelvalve nach dem ersten Aspekt der Erfindung. Weitere Vorteile und Merkmale der Erfindung sind dem Fachmann aus den beigefügten
Figuren und der detaillierten Beschreibung der Ausführungsbeispiele ersichtlich.
Fig. 1 zeigt ein erstes Ausführungsbeispiel der vorliegenden Erfindung in einer perspektivischen Ansicht;
Fig. 2 zeigt ein erstes Ausführungsbeispiel der vorliegenden Erfindung in einer Ansicht von der Seite;
Fig. 3 zeigt ein zweites Ausführungsbeispiel der vorliegenden Erfindung in einer perspektivischen Ansicht; und
Fig. 4 zeigt ein zweites Ausführungsbeispiel der vorliegenden Erfindung in einer perspektivischen Ansicht von der Rückseite.

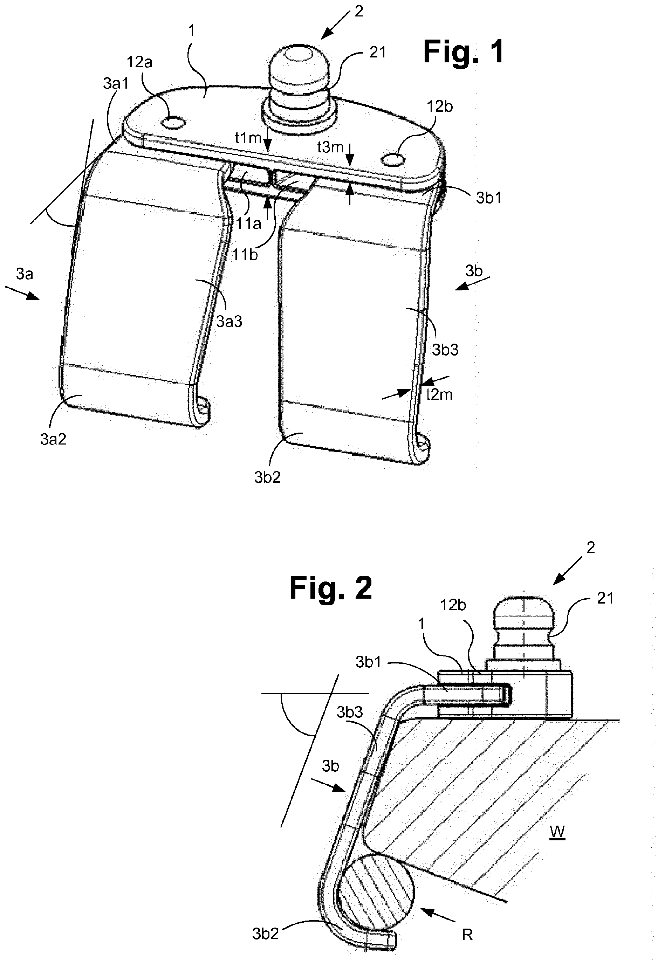

Ein erstes Ausführungsbeispiel der vorliegenden Erfindung ist unter Bezugnahme auf die Fig. 1 und 2 im Detail beschrieben. Dieses Ausführungsbeispiel bezieht sich auf eine Doppelvalve für einen Retraktor, welcher eingesetzt wird, um die Rippen eines Patienten im Seitenbereich aufzuspreizen

Bei dem ersten Ausführungsbeispiel der vorliegenden Erfindung ist die Doppelvalve für einen chirurgischen Retraktor ausgebildet mit einem Grundkörper 1, der einen im Wesentlichen zylindrischen Montagedorn 2 zur Montage der Doppelvalve an einem chirurgischen Retraktor aufweist. Der Montagedorn 2 erstreckt sich von der Oberfläche des Grundkörpers 1 nach oben. Darüber hinaus weist die Doppelvalve zwei Valvenelemente 3a, 3b auf. Der Montagedorn 2 hat eine kreiszylindrische Form mit einer umlaufenden Befestigungsnut 21, welche sich ungefähr auf halber Höhe des Montagedorns 2 befindet. Auf diese Weise hat der Montagedorn 2 in etwa die Form eines Pilzes. Bei diesem Ausführungsbeispiel weist die umlaufende Befestigungsnut 21 eine gleichbleibende Tiefe auf.

Die beiden Valvenelemente 3a, 3b sind mit ihren proximalen Enden 3a1, 3b1 in zwei getrennten Schlitzen 11a, 11b in dem Grundkörper 1 aufgenommen und an diesem mit Hilfe je eines Stiftes 12a, 12b drehbar befestigt. Die beiden Schlitze 11a, 11b sind in einer Seitenfläche des Grundkörpers 1 ausgebildet, welche bei der Verwendung der Doppelvalve der aufzuspreizenden Körperöffnung zugewandt ist. Beide Valvenelemente 3a, 3b weisen eine in Querrichtung ebene Innenfläche 3a4, 3b4 auf. Das freie distale Ende 3a2, 3b2 jedes Valvenelements 3a, 3b ist zu seiner Innenfläche 3a4, 3b4 hin umgebogen und bildet auf diese Weise eine Art Haken, der mit einer Rippe des Patienten in Eingriff gebracht werde kann und so ein Abrutschen der Valvenelemente 3a, 3b von der Rippe verhindert. Im vorliegenden Fall ist kein elastisches Element vorgesehen, welches die Valvenelemente 3a, 3b in eine Grundstellung drängt.

Die Verdrehung jedes Valvenelements 3a, 3b gegenüber dem Grundkörper 1 ist in beide Richtungen begrenzt. Der zwischen den beiden Schlitzen 11a, 11b vorhandene Steg begrenzt eine Verdrehung beider Valvenelemente 3a, 3b in Richtung zu dem jeweils anderen Valvenelement 3a, 3b hin, im Folgenden eine Drehung nach innen genannt. Da sich die beiden Schlitze 11a, 11b jeweils über die abgerundete Ecke des Grundelements 1 hinaus erstreckt, ist die Verdrehbarkeit nach außen größer als nach innen. Nichts desto trotz begrenzen die äußeren Seitenwände der beiden Schlitze eine Verdrehung des jeweiligen Valvenelements 3a, 3b nach außen. Allerdings kann aufgrund der breiten Öffnung der beiden Schlitze 11a, 11b auf Reinigungsöffnungen verzichtet werden.

Die proximalen Enden 3a1, 3b1 der beiden Valvenelemente 3a, 3b verlaufen parallel zu der Fläche des Grundkörpers 1. Der mittlere Bereich 3a3, 3b3 der beiden Valvenelemente 3a, 3b ist bei dem vorliegenden Ausführungsbeispiel um 70° gegenüber den proximalen Enden 3a1, 3b1 nach unten geneigt. Die Biegung der distalen Enden 3a2, 3b2 zu den Innenseiten 3a4, 3b4 der Valvenelemente hin, also von der zu spreizenden Körperöffnung weg, ist so gestaltet, dass die distalen Enden 3a2, 3b2 in etwa parallel zu den proximalen Enden 3a1, 3b1 verlaufen. Demnach beträgt die Biegung an den distalen Enden 3a2, 3b2 in etwa 110°.

Diese Neigung der mittleren Bereiche 3a3, 3b3 um 70° gegenüber dem Grundkörper stellt, wie dies in Fig. 2 gezeigt ist, einerseits einen festen und zuverlässigen Eingriff an den Rippen R des Patienten sicher und auf der anderen Seite ermöglicht er einen guten Einblick des Operateurs in die dann aufgespreizte Körperöffnung und somit auf das eigentliche Operationsgebiet. Dies wird auch dadurch erreicht, dass die mittleren Bereiche 3a3, 3b3 der Valvenelemente 3a, 3b das Weichgewebe W insbesondere im oberen Bereich der Fig. 2 seitlich wegdrücken.

Bei dem vorliegenden Ausführungsbeispiel sind zudem alle Bauteile aus Metall gefertigt. In diesem Fall wird eine Titan-Legierung verwendet. Der Einsatz von metallischen Werkstoffen ermöglicht eine schlanke Ausbildung der Valvenelemente und des Grundkörpers. Es können aber auch metallische Valvenelemente in Verbindung mit einem Grundkörper aus Kunststoff verwendet werden. Als Kunststoff eignet sich hier insbesondere PEEK (Polyetheretherketon), auch als Matrixmaterial in einem Verbundwerkstoff mit Kohlefasern oder anderen Fasern. Besonders vorteilhaft kann hier ein Hochleistungs-PEEK-Verbundwerkstoff mit einem Kohlefaseranteil von bis zu 60% eingesetzt werden. Als weiterer metallischer Werkstoff neben Titan und Titan-Legierungen eignet sich insbesondere gehärteter oder ungehärteter Edelstahl und entsprechende Legierungen. Die Valvenelemente können auch zunächst aus einem metallischen Werkstoff gefertigt werden und anschließend mit einem Kunststoff beschichtet oder umgossen werden, wobei sich hierzu neben den verschiedenen Formen von PEEK auch insbesondere Silikon eignet. Durch eine Beschichtung bzw. Umspritzung der metallischen Valvenelementkerne mit Kunststoff können Reflexionen verringert bzw. vermieden werden, was eine bessere Betrachtung des Operationsgebietes durch den Operateur ermöglicht.

Die erfindungsgemäße Doppelvalve wird frei drehbar in einer entsprechenden Aufnahme an einem Arm eines passenden Retraktors montiert. Gewöhnlich werden an beiden Armen des Retraktors identische Doppelvalven montiert. Nachdem der Operateur zwischen zwei Rippen eines Patienten eine Inzision gesetzt hat, werden beide Doppelvalven zwischen zwei benachbarte Rippen eingesetzt. Dabei hat der Operateur auf die korrekte Ausrichtung der Grundkörper der Doppelvalven und der Valvenelemente zu achten, sodass die Valvenelemente 3a, 3b mit ihrer Innenfläche 3a4, 3b4 an der jeweiligen Anlagefläche der Inzision anliegen und die Biegung der distalen Enden 3a2, 3b2 in Eingriff mit den Rippen gelangen. Anschließend wird der Retraktor aufgespreizt, um dem Operateur den Blick auf das Operationsgebiet und den Zugang zu diesem zu ermöglichen. Da die Rippen nicht gerade verlaufen sondern eine Krümmung aufweisen, verdrehen sich die beiden Valvenelemente 3a, 3b einer Doppelvalve so, dass ihre Querrichtung weitestgehend parallel zu der jeweiligen Anlagefläche verläuft. Genauer gesagt bildet die Querrichtung jedes Valvenelements 3a, 3b eine Tangente an eine konvexe Rippe bzw. Anlagefläche und eine Sekante an eine konkave Anlagefläche bzw. Rippe.

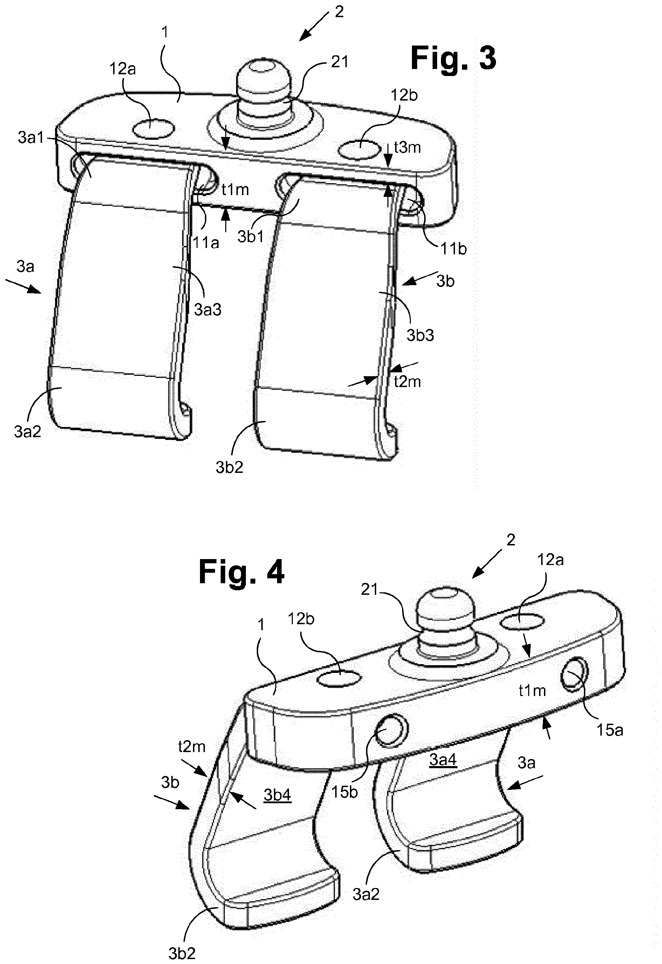

Im Folgenden ist ein zweites Ausführungsbeispiel der vorliegenden Erfindung unter Bezugnahme auf die Fig. 3 und 4 beschrieben.

Das zweite Ausführungsbeispiel hat zahlreiche Gemeinsamkeiten mit dem ersten Ausführungsbeispiel. Daher sind im Folgenden überwiegend die Unterschiede des zweiten Ausführungsbeispiels zu dem ersten Ausführungsbeispiel beschrieben.

Das zweite Ausführungsbeispiel ist vollständig aus Kunststoff gefertigt. Im vorliegenden Fall wird PEEK (Polyetheretherketon) verwendet. Da PEEK im Gegensatz zu Titan bzw. einer Titanlegierung eine geringere Festigkeit aufweist, sind die Bauteile des zweiten Ausführungsbeispiels dicker ausgebildet als bei dem ersten Ausführungsbeispiel. So ist sowohl die Gesamtdicke tm1 des Grundbauteils 1, die Dicke t3m der oberen und unteren Wand des Grundbauteils 1 sowie die Dicke t3m der Valvenelemente 3a, 3b des ersten Ausführungsbeispiels jeweils größer als die Dicke tk1, tk2, tk3 des entsprechenden Bauteils bei dem zweiten Ausführungsbeispiel. Auch der pilzförmige Montagedorn 2 hat aufgrund seiner größeren Dicke eine gedrungenere Form. Die Schlitze 11a, 11b weisen eine größere Höhe auf, um die dickeren proximalen Enden 3a1, 3b1 der beiden Valvenelemente 3a, 3b aufzunehmen. Die beiden Schlitze 11a, 11b haben zudem eine geringere Breite, sodass die Verdrehbarkeit der Valvenelemente 3a, 3b gegenüber dem ersten Ausführungsbeispiel stärker begrenzt ist. Der zwischen den Schlitzen 11a, 11b ausgebildete Steg ist breiter, um dem Grundkörper 2 insgesamt eine ausreichende Festigkeit zu geben. Zudem sind die äußeren Seitenwände der beiden Schlitze jeweils noch auf der vorderen Seitenfläche des Grundkörpers 2 angeordnet. Dies bedeutet, dass sich die Schlitze 11a, 11b nicht über die abgerundeten Ecken des Grundkörpers 2 hinaus in die angrenzenden Seitenflächen hinein erstrecken.

Bis auf die Dicke t2k der Valvenelemente 3a, 3b ist ihre Geometrie im Wesentlichen identisch zu der der Valvenelemente aus dem ersten Ausführungsbeispiel. Da auch die Stifte 12a, 12b, mit denen die proximalen Enden 3a1, 3b1 der Valvenelemente 3a, 3b in dem Grundkörper 2 drehbar befestigt sind, aus Kunststoff ausgebildet sind, weisen auch diese einen größeren Durchmesser auf. Die größere Dicke t2k der Valvenelemente 3a, 3b hat aber auch zur Folge, dass sich Verschmutzungen leichter in den Schlitzen 11a, 11b festsetzen können und schwerer zu entfernen sind. Daher sind, wie dies in der Fig. 4 gezeigt ist, zwei Reinigungsöffnungen 15a, 15b an der rückwärtigen Seitenfläche des Grundkörpers 1 vorgesehen. Jede Reinigungsöffnung 15a, 15b verbindet einen der Schlitze 11a, 11b mit der Umgebung und ermöglicht das Einführen eines Reinigungsfluids zu dem jeweiligen proximalen Ende 3a1, 3a2 des Valvenelements 3a, 3b, welches in dem Schlitz 11a, 11b aufgenommen ist. Besonders vorteilhaft ist bei diesem Ausführungsbeispiel, dass jeweils ein Schlitz 11a, 11b und die zugehörige Reinigungsöffnung 15a, 15b im Wesentlichen auf einer Linie liegen, sodass eine gute Strömung des Reinigungsfluids erzeugt werden kann, ohne dass sich Gebiete ausbilden, in denen die Strömung stark abnimmt und somit die Reinigung in diesen Gebieten nicht sicher gestellt werden kann. Für eine besonders leichte und gründliche Reinigung ist es auch vorteilhaft, wenn der Abstand zwischen der Innenwand der Schlitze 11a, 11b (also der Aufnahmeöffnungen) und dem proximalen Ende 3a1, 3b1 des jeweiligen Valvenelements 3a, 3b nicht zu gering ist.

In den Figuren nicht gezeigt ist ein Verbindungselement. Ein Verbindungselement ähnelt einem Grundkörper. An den stellen, an denen an einem Grundkörper die Stifte 12a, 12b für die Befestigung der Valvenelemente 3a, 3b vorgesehen sind, ist an einem Verbindungselement jeweils eine Montageaufnahme zur Aufnahme eines Montagedorns 2 einer Doppelvalve angeordnet. Außerdem sind an einem Verbindungselement keine Valvenelemente vorgesehen und der Abstand zwischen den beiden Montageaufnahmen ist größer als der Abstand der beiden Stifte 12a, 12b bei einer Doppelvalve. Ebenfalls in den Figuren nicht gezeigt ist eine Einzelvalve. Diese hat eine Form entsprechend einem Valvenelement, weist jedoch an ihrem proximalen Ende einen Montagedorn entsprechend dem Montagedorn 2 der Doppelvalve auf, um in einer Montageaufnahme eines Verbindungselements aufgenommen und drehbar befestigt zu werden. Die Länge der Einzelvalve kann größer als die eines Valvenelements sein, sodass die proximalen Biegungen der Einzelvalve mit denen der Valvenelemente einer Doppelvalve im wesentlichen auf derselben Höhe liegen, sodass ein gleichmäßiger Eingriff mit der jeweiligen Rippe des Patienten hergestellt werden kann.

Die vorliegende Erfindung ist vorstehend unter Bezugnahme auf einen Einsatz an den Rippen eines Patienten beschrieben, sie kann aber auch mit anderen Arten von chirurgischen Retraktoren eingesetzt werden, so beispielsweise für Retraktoren zum Einsatz am Sternum eines Patienten.

Vorstehend wurden Titan, Edelstahl, Legierungen mit diesen beiden Metallen, verschiedene Formen von PEEK und Silikon als Materialien genannt, die bei der Herstellung der erfindungsgemäßen Doppelvalven verwendet werden können. Prinzipiell kann aber jedes Metall Verwendung bei der Herstellung von erfindungsgemäßen Doppelvalven finden. Selbiges gilt für die genannten Kunststoffe. Über die genannten Kunststoffe hinaus können sämtliche Kunststoffe wie beispielsweise Elastomere verwendet werden. Die verschiedenen Metalle und Kunststoffe können auch beliebig geeignet kombiniert werden, um so Valvenelemente oder Grundkörper zu bilden. Auch die Befestigung der Valvenelemente an dem Grundkörper kann mittels verschiedenster Materialien gewährleistet werden.

Weitere Kombinationen der einzelnen Merkmale sind möglich und dem Fachmann ergeben sich aus dieser Beschreibung und den beigefügten Ansprüchen und Figuren zahlreiche weitere Modifikationen und Abwandlungen.

## Patentansprüche

1. Doppelvalve für einen chirurgischen Retraktor mit
einem Grundkörper (1), der einen im Wesentlichen zylindrischen Montagedorn (2) zur frei drehbaren Montage der Doppelvalve an einem chirurgischen Retraktor aufweist, und
genau zwei Valvenelementen (3a, 3b), die so an dem Grundkörper (1) angebracht sind, dass sie in einem gewissen Winkelbereich gegenüber dem Grundkörper (1) frei drehbar sind, wobei die Valvenelemente (3a, 3b) ein proximales Ende (3a1, 3b1) und ein freies distales Ende (3a2, 3b2) aufweisen,
**dadurch gekennzeichnet, dass**
der Grundkörper (1) eine Aussparung aufweist,
die Valvenelemente (3a, 3b) mit ihrem proximalem Ende (3a1, 3b1) in der Aussparung des Grundkörpers (1) aufgenommen und an dem Grundkörper (1) drehbar befestigt sind, und
die beiden Valvenelemente (3a, 3b) mit gleichem Hebelarm zu dem zylindrischen Montagedorn (2) vorgesehen sind.

2. Doppelvalve nach Anspruch 1, wobei der Montagedorn (2) so gestaltet ist, dass, wenn die Doppelvalve an einem chirurgischen Retraktor montiert ist, der Grundkörper (1) der Doppelvalve zumindest in einem vorgegebenen Winkelbereich gegenüber dem chirurgischen Retraktor frei drehbar ist.

3. Doppelvalve nach Anspruch 1 oder 2, wobei der Montagedorn (2) eine zumindest teilweise umlaufende Befestigungsnut (21) aufweist.

4. Doppelvalve nach Anspruch 3, wobei die Tiefe der Befestigungsnut (21) entlang ihrer Umlaufrichtung veränderlich ist.

5. Doppelvalve nach Anspruch 1, wobei der Grundkörper (1) in dem Bereich, in dem die proximalen Enden (3a1, 3b1) der Valvenelemente (3a, 3b) aufgenommen sind, Reinigungsöffnungen (15a, 15b) aufweist, um ein korrektes Reinigen und Sterilisieren der Doppelvalve sicher zu stellen.

6. Doppelvalve nach einem der vorstehenden Ansprüche, wobei mindestens ein Valvenelement (3a, 3b) eine quer zur Längsachse des Valvenelements (3a, 3b) konvex gekrümmte Innenfläche (3a4, 3b4) aufweist und/oder
mindestens ein Valvenelement (3a, 3b) ein zur Innenseite (3a4, 3b4) des Valvenelements gebogenes freies Ende (3a2, 3b2) aufweist.

7. Doppelvalve nach einem der vorstehenden Ansprüche, wobei zwischen mindestens einem Valvenelement (3a, 3b) und dem Grundkörper (1) ein elastisches Element vorgesehen ist, welches das mindestens eine Valvenelement (3a, 3b) in eine vordefinierte Grundstellung drängt.

8. Doppelvalve nach einem der vorstehenden Ansprüche, wobei mindestens ein Valvenelement (3a, 3b) eine weiche Beschichtung oder Polsterung an seiner Innenfläche (3a4, 3b4) aufweist.

9. Doppelvalve nach einem der vorstehenden Ansprüche, wobei die Verdrehung mindestens eines Valvenelements (3a, 3b) gegenüber dem Grundkörper (1) begrenzt ist.

10. Doppelvalve nach einem der vorstehenden Ansprüche, wobei die Längsachse jedes Valvenelements (3a, 3b) in einem Winkel (a) zwischen 50° und 90°, vorzugsweise zwischen 60° und 80°, und insbesondere zwischen 65° und 75° gegenüber einer Ebene geneigt ist, die sich senkrecht zu den Drehachsen der Valvenelemente erstreckt.

11. Chirurgischer Retraktor mit
einer Doppelvalve nach einem der Ansprüche 1 bis 10.

## Claims

1. Double blade for a surgical retractor, with
a base body (1), which has a substantially cylindrical mounting mandrel (2) for mounting the double valve in a freely rotatable manner on a surgical retractor, and
exactly two blade elements (3a, 3b), which are mounted on the base body (1) such that they are freely rotatable with respect to the base body (1) within a certain angular range, wherein the blade elements (3a, 3b) have a proximal end (3a1, 3b1) and a free distal end (3a2, 3b2),
**characterized in that**
the base body (1) has a recess,
the blade elements (3a, 3b) are received with their proximal end (3a1, 3b1) in the recess of the base body (1) and are fastened rotatably on the base body (1), and
the two blade elements (3a, 3b) are provided with the same lever arm to the cylindrical mounting mandrel (2).

2. Double blade according to Claim 1, wherein the mounting mandrel (2) is configured such that, when the double blade is mounted on a surgical retractor, the base body (1) of the double blade is freely rotatable with respect to the surgical retractor at least within a predetermined angular range.

3. Double blade according to Claim 1 or 2, wherein the mounting mandrel (2) has an at least partially circumferential fastening groove (21).

4. Double blade according to Claim 3, wherein the depth of the fastening groove (21) is variable along its circumferential direction.

5. Double blade according to Claim 1, wherein the base body (1), in the region in which the proximal ends (3a1, 3b1) of the blade elements (3a, 3b) are received, has cleaning openings (15a, 15b) in order to ensure correct cleaning and sterilization of the double blade.

6. Double blade according to one of the preceding claims, wherein at least one blade element (3a, 3b) has a convexly curved inner face (3a4, 3b4) transverse to the longitudinal axis of the blade element (3a, 3b), and/or
at least one blade element (3a, 3b) has a free end (3a2, 3b2) bent towards the inner side (3a4, 3b4) of the blade element.

7. Double blade according to one of the preceding claims, wherein an elastic element is provided between at least one blade element (3a, 3b) and the base body (1), which elastic element pushes the at least one blade element (3a, 3b) into a predefined basic position.

8. Double blade according to one of the preceding claims, wherein at least one blade element (3a, 3b) has a soft coating or padding on its inner face (3a4, 3b4).

9. Double blade according to one of the preceding claims, wherein the rotation of at least one blade element (3a, 3b) with respect to the base body (1) is limited.

10. Double blade according to one of the preceding claims, wherein the longitudinal axis of each blade element (3a, 3b) is inclined at an angle (a) of between 50° and 90°, preferably of between 60° and 80°, and in particular of between 65° and 75°, with respect to a plane extending perpendicularly to the axes of rotation of the blade elements.

11. Surgical retractor with
a double blade according to one of Claims 1 to 10.

## Revendications

1. Double valve pour un écarteur chirurgical, comprenant
un corps de base (1) qui présente un mandrin de montage (2) essentiellement cylindrique pour le montage librement rotatif de la double valve sur un écarteur chirurgical, et
exactement deux éléments de valve (3a, 3b) qui sont montés sur le corps de base (1) de telle sorte qu'ils puissent tourner librement sur une certaine plage angulaire par rapport au corps de base (1), les éléments de valve (3a, 3b) présentant une extrémité proximale (3a1, 3b1) et une extrémité distale libre (3a2, 3b2), **caractérisée en ce que**
le corps de base (1) présente un évidement,
les éléments de valve (3a, 3b) sont reçus avec leur extrémité proximale (3a1, 3b1) dans l'évidement du corps de base (1) et sont fixés de manière rotative sur le corps de base (1), et
les deux éléments de valve (3a, 3b) sont prévus avec le même bras de levier par rapport au mandrin de montage cylindrique (2).

2. Double valve selon la revendication 1, dans laquelle le mandrin de montage (2) est configuré de telle sorte que lorsque la double valve est montée sur un écarteur chirurgical, le corps de base (1) de la double valve puisse tourner librement au moins sur une plage angulaire prédéfinie par rapport à l'écarteur chirurgical.

3. Double valve selon la revendication 1 ou 2, dans laquelle le mandrin de montage (2) présente une rainure de fixation (21) au moins en partie périphérique.

4. Double valve selon la revendication 3, dans laquelle la profondeur de la rainure de fixation (21) varie le long de sa direction périphérique.

5. Double valve selon la revendication 1, dans laquelle le corps de base (1), dans la région dans laquelle sont reçues les extrémités proximales (3a1, 3b1) des éléments de valve (3a, 3b), présente des ouvertures de nettoyage (15a, 15b) afin de garantir un nettoyage et une stérilisation corrects de la double valve.

6. Double valve selon l'une quelconque des revendications précédentes, dans laquelle au moins un élément de valve (3a, 3b) présente une surface interne (3a4, 3b4) de courbure convexe transversalement à l'axe longitudinal de l'élément de valve (3a, 3b) et/ou
au moins un élément de valve (3a, 3b) présente une extrémité libre (3a2, 3b2) cintrée vers le côté interne (3a4, 3b4) de l'élément de valve.

7. Double valve selon l'une quelconque des revendications précédentes, dans laquelle entre au moins un élément de valve (3a, 3b) et le corps de base (1) est prévu un élément élastique qui pousse l'au moins un élément de valve (3a, 3b) dans une position de base prédéfinie.

8. Double valve selon l'une quelconque des revendications précédentes, dans laquelle au moins un élément de valve (3a, 3b) présente un revêtement souple ou un rembourrage sur sa surface interne (3a4, 3b4).

9. Double valve selon l'une quelconque des revendications précédentes, dans laquelle la rotation d'au moins un élément de valve (3a, 3b) par rapport au corps de base (1) est limitée.

10. Double valve selon l'une quelconque des revendications précédentes, dans laquelle l'axe longitudinal de chaque élément de valve (3a, 3b) est incliné suivant un angle (α) compris entre 50° et 90°, de préférence compris entre 60° et 80°, et en particulier compris entre 65° et 75° par rapport à un plan qui s'étend perpendiculairement aux axes de rotation des éléments de valve.

11. Écarteur chirurgical comprenant une double valve selon l'une quelconque des revendications 1 à 10.
